Europäisches Patentamt

European Patent Office

Office européen des brevets

(11) Publication number: **0 233 289 B1**

# EUROPEAN PATENT SPECIFICATION

(45) Date of publication of patent specification: **10.03.93**

(51) Int. Cl.5: **C07K 15/00**, C12P 21/00, C12N 5/00, C12N 15/00, A61K 39/40, //(C12P21/00, C12R1:91)

(21) Application number: **86900258.4**

(22) Date of filing: **20.12.85**

(86) International application number: **PCT/JP85/00698**

(87) International publication number: **WO 86/03754 (03.07.86 86/14)**

(54) **HYBRIDOMAS PRODUCING ANTI-PSEUDOMONAS AERUGINOSA HUMAN MONOCLONAL ANTIBODY.**

(30) Priority: **26.12.84 JP 273155/84**
**26.12.84 JP 273156/84**
**28.12.84 JP 274659/84**

(43) Date of publication of application:
**26.08.87 Bulletin 87/35**

(45) Publication of the grant of the patent:
**10.03.93 Bulletin 93/10**

(84) Designated Contracting States:
**AT BE CH DE FR GB IT LI NL SE**

(56) References cited:
**EP-A- 0 057 107      EP-A- 0 101 039**
**EP-A- 0 105 804      EP-A- 0 163 493**
**WO-A-84/04458      WO-A-85/01659**

(73) Proprietor: **TEIJIN LIMITED**
**11 Minamihonmachi 1-chome Higashi-ku**
**Osaka-shi Osaka 541(JP)**

(72) Inventor: **SAWADA, Shuzo**
**15-6, Tamadaira 5-chome**
**Hino-shi Tokyo 191(JP)**
Inventor: **KAWAMURA, Takashi**
**Tama House No. 1 Tamadaira 3-chome**
**Hino-shi Tokyo 191(JP)**
Inventor: **MASUHO, Yasuhiko**
**20-2, Tamadaira 5-chome**
**Hino-shi Tokyo 191(JP)**
Inventor: **TOMIBE, Katsuhiko**
**17-3, Kaiigusa 1-chome**
**Suginami-ku Tokyo 167(JP)**

(74) Representative: **Votier, Sidney David et al**
**CARPMAELS & RANSFORD 43, Bloomsbury Square**
**London WC1A 2RA (GB)**

EP 0 233 289 B1

EP 0 233 289 B1

Chemical Abstracts, volume 103, no. 23, 9 December 1985, Columbus, Ohio, (US), S. Wawada et al.: "Characterization of a human monoclonal antibody to lipopolysaccharides of Pseudomonas aeruginosa serotype 5; a possible candidate as an immunotherapeutic agent for infections with P. aeruginosa", see page 550, abstract no. 194664h & J. Infect. Dis 1985, 152(5), 965-70

Proc. Natl. Acad. Sci. USA, volume 82, March 1985, N.N.H. Teng et al.: "Protection against Gram-negative bacteremia and endotoxemia with human monoclonal IgM antibodies", pages 1790-1794, see page 1790, left-hand column, lines 1-7, 9-20 and line 40 - right-hand column, line 7; page 1791, left-hand column, lines 11-13; page 1792, right-hand column, lines 22-30; page 1793, left-hand column, line 1 - right-hand column, line 5, lines 9-14; table 4 page 1794, left-hand column, table 5, liens 2-9, 12-15 and right-hand column, lines 17-24

Chemical Abstracts, volume 101, no. 25, 17 December 1984, Columbus, Ohio, (US), S. Sawada et al.: "Protection against infection with Pseudomonas aeruginosa by passive transfer of monoclonal antibodies to lipopolysaccharides and outer membrane proteins", see page 586, abstract no. 228662b & J. Infect. Dis. 1984, 150(4), 570-6

Chemical Abstracts, volume 101, no. 17, 22 October 1984, Columbus, Ohio, (US), L.M. Mutharia et al.: "Monoclonal antibodies specific for Esherichia coli J5 lipopolysaccharide: cross-reaction with other Gram-negative bacterial species", see page 532, abstract no. 149444j & Infect. Immun. 1984, 45(3), 631-6

## Description

Technical Field

The present invention relates to human monoclonal antibodies which act against Pseudomonas aeruginosa, mouse-human hybridomas and transformed human cells which produce the same, and processes for producing them. The object of this invention is to provide anti-P. aeruginosa human monoclonal antibodies which are useful for diagnosis and therapy of patients with P. aeruginosa infections. Another object of this invention is to provide mouse-human hybridomas and transformed human cells which produce such anti-P. aeruginosa human monoclonal antibodies efficiently.

Background Art

P. aeruginosa was originally known as a bacterium having low potentialities to cause diseases; however, as a result of the encouraged replacement of bacteria due to the administration of antibiotics, infectious diseases produced by antibiotic-resistant P. aeruginosa have become prevalent and patients with immune deficiency disease are often found. Especially in the cases of patients with cystic fibrosis, thermal burns and cancer, the diseases are frequently accompanied by the onset of worst symptoms. These infectious diseases involve the problem that the use of antibiotics in their therapy and cure is not always effective since P. aeruginosa has often been made antibiotic-resistant. Accordingly, so-called immunotherapy conducted by use of anti-P. aeruginosa antibodies has aroused interest which has stimulated the study thereof but the results have not yet been clinically accepted. There is another problem that, though a proper remedy for infectious diseases arising from P. aeruginosa indispensably requires accurate diagnosis in their early stages, the conventional methods in which anti-serum is used do not necessarily afford satisfactory data. It is, therefore, necessary for us to have anti-P. aeruginosa monoclonal antibodies to solve these problems.

As the surface antigens of P. aeruginosa, lipopolysaccharide (LPS), outer membrane protein (OMP), flagella, and slime are generally known. Of these, LPS has several types of O-polysaccharide side chains which determine the serotype of P. aeruginosa and 16 serotypes ranging from type 1 to type 16 have hitherto been made known (according to Homma's classification). Besides the O-polysaccharide side chains, LPS also has a core region and lipid A. The lipid A is buried beneath the outer membrane of P. aeruginosa and the core region extends outward from the lipid A beyond the outer membrane through the medium of 2-keto-3-deoxyoctonate. Furthermore, the O-polysaccharide side chain extends outward from the core region. It has also been reported that antibodies to LPS are readily formed in human bodies and animals and function protectively against infections. It is said that the antibody to LPS binds to the LPS of P. aeruginosa, and that the resulting antigen-antibody conjugate is fixed to complement so that P. aeruginosa may suffer immuno-bacteriolysis or may be destroyed by such a phagocyte as polymorphonuclear leukocyte, etc., thus allowing the carrier of P. aeruginosa to escape from its infection. A patient infected with P. aeruginosa is inclined to have more P. aeruginosa antigens and less LPS antibodies. To prevent the development of this symptomatic characteristic and to cure a patient of P. aeruginosa infection, IgG preparations produced from human blood have hitherto been used; however, the antibody titer of P. aeruginosa contained in the preparation is too low to display enough clinical potency to cure the infections.

On the other hand, it is a known method in which the technique of cell fusion is utilized to fuse a lymphocyte or a B cell (antibody-producing cell), which produces a specific antibody but is destined to perish in due course of time, and a myeloma cell, which never stops proliferating in the culture tube, so that a hybridoma (hybrid cell) strain, which continues to produce and secrete specific antibodies, may be formed. Monoclonal antibodies, which are produced by the hybridoma thus prepared, have a number of miscellaneous applications such as pure chemical reagents of high accuracy and reliability including test reagents, labelling reagents, and affinity chromatography impregnants, and are also expected to be used as therapeutic and prophylactic drugs for various diseases.

It is accepted as a general consideration that monoclonal anti-P. aeruginosa antibodies can be obtained by preparing an anti-P. aeruginosa antibody-producing hybridoma by fusing an anti-P. aeruginosa antibody-producing cell and a myeloma cell. To describe more concretely, Japanese Patent Laid-Open No. 29622/'84, for instance, discloses that an anti-P. aeruginosa mouse antibody-producing monoclonal mouse-mouse hybridoma was obtained by fusing spleen cells (antibody-producing cells) of a BALB/C mouse immunized with a LPS of P. aeruginosa and myeloma cells (P3-X 63-Ag 8-UI strain) of a mouse to give a hybridoma, which was then subjected to cloning to obtain an anti-P. aeruginosa mouse antibody-producing monoclonal mouse-mouse hybridoma, and that the obtained mouse monoclonal antibodies showed a

3

preventive effect against mouse P. aeruginosa infections.

As described in the above, with regard to anti-P. aeruginosa antibodies, a single successful concrete example has been offered by the realization of an anti-P. aeruginosa mouse monoclonal antibody. In making a diagnosis and performing a cure for the infectious diseases of man, the use of anti-P. aeruginosa human antibody of homologous protein is more useful and safer, but this requires the establishment of a mouse-human hybridoma or a human-human hybridoma which requires the use of human antibody producing cells. The successful establishment of such hybridomas is difficult due to the problems of collecting and controlling suitable antibody-producing human cells properly, since in the case of human beings, unlike the case of animals, no method is allowed to be taken so far as it involves the immunization of human beings with a great amount of P. aeruginosa or with their surface antigens, the collection of effectively stimulated antibody-producing cells, and their use for effecting the cell fusion.

The European Application EP-A-0 163 493 filed on 23/05/85 and published on 04/12/85 discloses human monoclonal antibodies specifically reacting with different Pseudomonas aeruginosa Fisher immunotypes. This document falls under Article 54 (3) EPC.

Disclosure of Invention

As the result of an intensive study of mouse-human hybridomas in an effort to obtain anti-P. aeruginosa human antibodies, the present inventors have successfully come to obtain specific anti-P. aeruginosa antibodies by cell fusion and transformation.

The present invention relates to the use as diagnostic reagents of anti-P. aeruginosa human IgG monoclonal antibodies which have the property of recognizing P. aeruginosa's lipopolysaccharide (consisting of an O-polysaccharide side chain, core region, and lipid A). The monoclonal antibodies recognize the O-polysaccharide side chain of the lipopolysaccharide whose serotype is any of types 1, 2, 5, 7, 8, 10 and 13.

The invention provides a mouse-human hybridoma or cell line arising therefrom which produces human anti-P. aeruginosa monoclonal antibodies having specificity for an O-polysaccharide chain of lipopolysaccharide, whose serotype is any of types 1, 2, 5, 7, 8, 10 and 13, wherein said hybridoma is obtained by:
(a) sensitizing lymphocytes collected from a tonsil or spleen by mitogen;
(b) selecting anti-P. aeruginosa antibody-producing human cells;
(c) fusing said antibody-producing cells with mouse myeloma cells to produce hybridomas;
(d) identifying and isolating hybridomas which produce said human anti-P. aeruginosa monoclonal antibodies; and
(e) cloning and culturing said hybridomas obtained in said step (d).

Also included in the invention is a process for producing a mouse-human hybridoma which produces anti-P. aeruginosa human monoclonal antibodies having specificity for an O-polysaccharide chain of lipopolysaccharide, whose serotype is any of types 1, 2, 5, 7, 8, 10 and 13, comprising (a) sensitizing lymphocytes collected from a tonsil or spleen by mitogen; selecting and collecting tissues which contain many anti-P. aeruginosa antibody-producing cells from among antibody-producing human cellular tissues and then fusing the cells of such human tissues with mouse myeloma cells.

Best Mode of Carrying out the Invention

What is referred to as an antibody-producing human cell is a lymphocyte (or B cell) of human beings, which is a cell that secretes or is capable of secreting antibodies. This lymphocyte is contained in the cells of the spleen, lymph nodes, peripheral blood, bone marrow, tonsils, and adenoids.

For the object of this invention, a lymphocyte collected from the tonsils or the spleen is used.

An explanation will first be made for the cell fusion method of the two methods proposed by the present invention.

As the mouse myeloma cells to be fused with antibody-producing cells, cell lines resistant to 8-azaguanine are advantageously used, and there are BALB/C mouse's P3-X63-Ag8, P3-X63-Ag8-UI P3-NSI/I-Ag 4-1, P3-X63-Ag8-6.5.3, SP2/0-Ag14, FO, and MPCII-45.6TG1.7 known publicly.

In this invention, human tissue which contains many anti-P. aeruginosa antibody-producing cells is selected and these cells of human tissue are allowed to fuse with mouse myeloma cells. The process involves the selection of P. aeruginosa having a specific LPS as the antigen. A separate lot of mononuclear cells are prepared by sensitizing lymphocytes collected from a human tonsil or a spleen by mitogen. The mononuclear cells thus prepared are cultivated for 5 to 7 days under conditions in which such mitogen as pokeweed mitogen is used in an incubator under 5% $CO_2$ and the antibodies contained in the supernatant of the culture liquid are determined with the use of plates fixed with the aforementioned P. aeruginosa to pick up the systems which contain the desirable mononuclear cells. The lymphocytes of these systems and the myeloma cells are made to fuse together to form heterogenous colonies of hybridomas. The cell fusion can be effected by a known method. For instance, a mixture of antibody producing cells and myeloma cells is prePared at a mixing ratio of 10:1 to 1:10, preferably at a ratio of 1:1 to 1:3. A suitable cell fusion agent such as RPMI 1640, which contains about 35% polyethylene glycol (molecular weight of about 1,000 to 6,000) and about 7.5% dimethyl sulfoxide, is added to the mixture and the mixture is stirred at room temperature to 37°C for 1 to several minutes. Thereafter, the mixture is diluted with RPMI 1640 containing 10% FCS, washed, and adjusted with hypoxanthine-aminopterin-thymidine (HAT) selective culture medium so that the cell concentration may be in the range of 1 to $5X10^5$ cells/ml. This is delivered, for instance, into 96-well plates in an amount of 0.2 ml per well and the plates are left standing in the air containing 5% $CO_2$ at 35 to 38°C for 2 to 3 weeks for cultivation. In the HAT culture medium, only hybridomas can survive and myeloma cells and fused cells between myelomas resistant to 8-azaguanine can not survive (whereas antibody producing cells left unfused perish in a few days). Then selections are made from these hybridoma colonies to obtain those hybridomas only that secrete human monoclonal antibodies specific to LPS of P. aeruginosa. This selection process (or screening) can be carried out by subjecting human monoclonal antibodies produced from different hybridomas to enzyme immunoassay by use of plates fixed with P. aeruginosa having the desired serotype of LPS of such P. aeruginosa. To obtain human monoclonal antibodies reactive to all serotypes of P. aeruginosa, 16 kinds of P. aeruginosa of different serotypes must be used. These kinds of P. aeruginosa are available from the American Type Culture Collection (ATCC).

The desired hybridomas which secrete monoclonal antibodies to P. aeruginosa must then be made into clonal cells by a cloning process. To mention a concrete example, this process can be achieved by applying a soft agar culture method. After cultivation in the soft agar for about 2 to 3 weeks, the grown colonies are collected and selected by subjecting them to enzyme immunoassay to determine their antibody activity against P. aeruginosa. The selected hybridomas are then cultured to effect the production of the desired human monoclonal antibodies specific to LPS.

The anti P. aeruginosa human antibody-producing mouse-human hybridomas which have been selected by cloning may be preserved by freezing or can be mass-produced by culture according to a suitable method. Thus produced cell lines or facsimiled cells are included in the scope of the present invention. Also, mutant lines arising from said hybridomas are included in the scope of this invention so far as such mutants produce substantially the Same anti-P. aeruginosa human antibodies as those produced by the cloned hybridomas.

Monoclonal anti-P. aeruginosa human antibodies which specifically bind to P. aeruginosa LPS can be obtained from the supernatant of the medium used for culturing the hybridomas and the transformed cells. Anti-P. aeruginosa human antibodies can also be obtained from the ascites or the blood serum of an animal by transplanting such cells. The purification of anti P. aeruginosa human antibodies can be carried out by such a method as affinity chromatography conducted with the use of monoclonal antibodies.

The anti-P. aeruginosa LPS human monoclonal antibodies bind specifically to P. aeruginosa LPS and work in cooperation with the complement components to subject P. aeruginosa to immune bacteriolysis. These human monoclonal antibodies can also protect the mice against the infections in the model experimental system of P. aeruginosa infected mice.

Every type of human monoclonal antibody produced in the process of this invention can be mixed with the others and be allowed to bind to any of the whole 16 serotypes of P. aeruginosa. Anti P. peruginosa LPS human monoclonal antibodies bind to specific serotype to exercise the protective functions against infections in model infection experiments. More particularly, a human monoclonal antibody which is specific to a certain serotype binds to P. aeruginosa of the same serotype up to 100% but fails completeiy to bind to P. aeruginosa of a different serotype. Human monoclonal antibodies can, therefore, be bound to P. aeruginosa of all presently known serotypes by mixing all types of human monoclonal antibodies obtained.

The anti-P. aeruginosa human monoclonal antibodies which recognize LPS of P. aeruginosa can be formulated into medical preparations together with other commonly used medical additives such as glycine or mannitol for use as a remedy for curing human beings and animals with P. aeruginosa infections. In making such medical preparations, it is not always necessary to mix every type of monoclonal antibody corresponding to all serotypes. If the preparations are made to contain the anti-P. aeruginosa human monoclonal antibodies corresponding in serotype to P. aeruginosa of type 1, type 5, type 7, type 8 and type 10 at the least, such preparations can cure more than 70% of P. aeruginosa infections.

The dosage regimen of the human monoclonal antibodies differs depending upon the states of the respective patients including the conditions of P. aeruginosa infections to be treated and also upon the dose and the frequency of administration. Prior to their administration, antibodies are generally mixed with a pharmaceutically permissible innoxious carrier substance such as isotonic sodium chloride solution, and are then given to a patient by any medically suitable method such as intravenous injection. It is a general practice to make antibodies exist in the carrier at a concentration of about 0.5 $\mu$g antibodies/ml to 500 $\mu$g antibodies/ml. The dose of antibodies to be administered generally ranges from 10 $\mu$g to 1 mg per kg of body weight at a time. Successive administrations are made, as the case may require.

The present invention will now be illustrated, but is not intended to be limited, by the following examples.

Example 1

(1) Selection of anti-P. aeruginosa antibody-producing cells

Eight lots of tonsillar cells were obtained from 8 patients with tonsillitis and mononuclear cells were prepared from them by use of Ficoll-Paque®. The mononuclear cells were then made to float in medium A (consisting of RPMI 1640 + 10% fetal bovine serum + 2 mM L-glutamine + 1 mM sodium pyruvate + 0.02 mg/ml L-serine + 80 $\mu$g/ml gentamicin) at a concentration of 5 X $10^6$ cells/ml, and pokeweed mitogen (PWM) was added thereto in an amount of 20 $\mu$g/ml. This specimen was dispensed into culture plates, each containing 200 $\mu$l, and cultured for 6 to 7 days in a 5% $CO_2$ incubator. Thereafter, 100 $\mu$l each of the respective culture supernatants were transferred to the plates coated with P. aeruginosa and the determination was conducted by enzyme immunoassay with the result that only one lot was found producing anti-P. aeruginosa antibodies prolifically. The mononuclear cells of this lot were used in the cell fusion with mouse myeloma P3-X63-Ag8-UI (hereinafter referred to as P3UI).

(2) Cell fusion

P3UI was cultured beforehand in medium A. The cell concentration at the time of cell fusion was 6 X $10^5$ cells/ml. The abovementioned lot of tonsillar lymphocytes which showed an excellent anti-P. aeruginosa antibody productivity and P3UI were washed twice respectively with serum-free RPMI 1640 medium. The lymphocytes and P3UI cells (5 X $10^6$) were placed together in a test tube, centrifuged at a speed of 1,500 r.p.m. for 5 minutes, and the supernatant was discarded. The cell pellets were thoroughly dispersed by tapping the test tube. Then 0.5 ml of a polyethylene glycol solution (5.75 ml of RPMI 1640 + 3.5 ml of polyethylene glycol 1,500 + 0.75 ml of dimethyl sulfoxide) (hereinafter referred to as PEG solution) was added thereto to allow the cells to float gently. One minute later 1 ml of RPMI 1640, another 2 minutes later 4 ml of HAT medium (RPMI 1640 + 20% fetal bovine serum + 80 $\mu$g/ml gentamicin + 95 $\mu$M hypoxanthine + 0.4 $\mu$M aminopterin + 1.6 $\mu$M thymidine), and further 2 minutes later 4 ml of HAT medium were respectively added in this order. Finally 25 ml of HAT medium was added to make 25 ml of cell-floating solution. This was dispensed into a 96-well culture plate and cultured at 37°C in the air containing 5% $CO_2$. Half of the medium was changed with a new HT medium (one with A excluded from HAT) until hybridomas were obtained.

(3) Cloning and culture

The hybridoma culture supernatants were tested by ELISA with the use of plates coated with formalin-killed P. aeruginosa to obtain human monoclonal antibody P3 which binds to P. aeruginosa serotype, Homma type 5. Then the P3-producing hybridomas were cloned twice by limiting dilution (with the use of 2 pieces of 96-well plates), and thus mouse-human hybridomas which produce human monoclonal antibodies (IgG, λ) that is called P3D9, were finally obtained.

The obtained hybridomas were cultured in 1 ℓ of serum-free RDF/TES medium (see Proc. Natl. Acad. Sci. U.S.A., vol. 79, pp 1158~1162) and the culture which contained P3D9 was concentrated by use of ultrafiltration membrane PM 30 (by Amicon) to 30 ml in volume. This concentrate was subjected to column chromatography on DEAE-Sephacel® to give 12 mg of purified P3D9.

(4) Protective activity of monoclonal antibodies against infections

P. aeruginosa strains N-2 and 97 isolated from clinical specimens were selected to study the protective effect of P3D9 against infections. The serotype of strain N-2 was Homma type 5 and that of strain 97 was Homma type 7.

Groups of 10 strain ICR mice (male, 4 weeks old, weighing 16 to 20g) were injected intra-peritoneally with 2 or $4LD_{50}$ of P. aeruginosa strain N-2 or strain 97. One hour after injection the animals were injected with 1 $\mu$g or 10 $\mu$g of P3D9. The positive control mice were given IgG prepared from human serum and the negative control mice were given normal saline solution likewise. The results obtained from the 5-day observations are shown in Table 1.

Table 1

| Antibody | P. aeruginosa strain : Dose | | |
|---|---|---|---|
| | N-2 $2LD_{50}$ | N-2 $4LD_{50}$ | 97 $2LD_{50}$ |
| | * | | |
| P3D9 10 $\mu$g/mouse | 10/10 | 5/10 | 0/10 |
| P3D9 1 $\mu$g/mouse | 10/10 | 0/10 | 0/10 |
| Human IgG 1 mg/mouse | 10/10 | 5/10 | 9/10 |
| Normal saline solution | 0/10 | 0/10 | 0/10 |

* Survivors/Experimental subjects

It is apparent from Table 1 that human monoclonal antibody P3D9 of this invention shows the protective activity specific to strain N-2 (Homma type 5) of P. aeruginosa.

Example 2

(1) Selection of antibody-producing cells and cell fusion were conducted following the same procedure as taken in Example 1 except for the use of human spleen cells. The obtained hybridoma supernatant was transferred to the plates coated with formalin-killed P. aeruginosa and subjected to ELISA, the result of which showed that there were human monoclonal antibodies B8 capable of binding to any of P. aeruginosa serotypes, Homma type 2, Homma type 7, and Homma type 13. The B8 producing hybridoma was cloned two times by limiting dilution to give mouse-human hybridoma which produces human monoclonal antibody (IgG, $\lambda$) called B8E2.

This hybridoma was cultured in 1 $\ell$ of RPMI 1640 with 10% FCS added thereto and B8E2 was recovered from the culture supernatant by precipitation with ammonium sulfate (final saturation, 50%), followed by partial purification with DEAE-Sephacel®. The determination of human Ig was made by single radial immunodiffusion method (SRID) and 8 mg of an antibody solution containing B8E2.

It has been confirmed upon ELISA conducted with the use of plates coated with LPS extracted from Homma type 2, Homma type 7 and Homma type 13 by Johnson and Perry's method (Can. J. Microbiol., 1976, vol. 22, pp 29 to 34) and heat-treated LPS (at 100°C for 30 minutes) that the obtained monoclonal antibody combines to each of said LPS and it has also been found that it is a monoclonal antibody which can recognize LPS.

(2) Protective activity of monoclonal antibodies against infections

The protective activity of B8E2 was studied according to the same method as Example 1, wherein P. aeruginosa strains N-2 (Homma type 5) and 97 (Homma type 7) isolated from clinical specimens were used. The result is shown in Table 2.

Table 2

| Antibody | P. aeruginosa strain : Dose | | |
|---|---|---|---|
| | 97 $2LD_{50}$ | 97 $4LD_{50}$ | N-2 $2LD_{50}$ |
| B8E2 10 $\mu$g/mouse | 10/10 | 10/10 | 0/10 |
| B8E2 1 $\mu$g/mouse | 10/10 | 8/10 | 0/10 |
| Human IgG 1 mg/mouse | 10/10 | 10/10 | 10/10 |
| Normal saline solution | 0/10 | 0/10 | 0/10 |

It is clearly shown by Table 2 that human monoclonal antibody B8E2 of the present invention shows a specific protective effect against infections with P. aeruginosa strain 97 (Homma type 7).

Example 3

Selection of antibody producing cells and cell fusion were carried out by the use of human tonsillar cells according to Example 1. The obtained hybridoma supernatant was added to plates coated with formalin-killed P. aeruginosa and subjected to ELISA for determination, whose result gave 31-7 to bind to P. aeruginosa serotype Homma type 1, 31-8 to bind to Homma type 7, 31-9 to bind to Homma type 8, and 31-12 to bind to Homma type 10. The hybridomas productive of these human monoclonal antibodies were cloned twice by limiting dilution and agar dilution (by use of five 96-well plates) to obtain hybridomas productive of human IgG of 31-7-2A, 31-8-5G, 31-9-F4, and 31-12-H3 respectively.

Following the procedure of Example 2, the obtained hybridomas were cultured, partially purified, and studied by ELISA to see how they would bind to LPS and heat-treated (100°C X 2 hrs) LPS in order to determine the antigen binding site to which the respective monoclonal antibodies are expected to react. The result is shown in Table 3 (reaction time, 60 minutes).

## Table 3

| | Homma type 1 | | Homma type 7 | | Homma type 10 | | Homma type 8 | |
|---|---|---|---|---|---|---|---|---|
| | LPS | Heat-treated LPS | LPS | Heat-treated | LPS | Heat-treated LPS | LPS | Heat-treated LPS |
| 31-7-2A | 1.5* | 1.5 | 0 | 0 | 0 | 0 | 0 | 0 |
| 31-8-5G | 0 | 0 | 1.3 | 1.2 | 0 | 0 | 0 | 0 |
| 31-12-H3 | 0 | 0 | 0 | 0 | 1.1 | 1.1 | 0 | 0 |
| 31-9-F4 | 0 | 0 | 0 | 0 | 0 | 0 | 1.2 | 1.2 |

**\* Figures in the above table indicate ELISA scores.**

Example 4

The binding spectrum of human monoclonal antibodies, produced by mouse-human hybridomas according to Examples 1 to 3, to P. aeruginosa, other Pseudomonas species, the strains of Enterobacteriaceas, and other gram-positive strains were determined by ELISA. The result is shown in Table 4.

8

Table 4

| P. aeruginosa strain or gram-positive bacterial strain | Serotype | ELISA score (60 min. after starting) | | | | | |
|---|---|---|---|---|---|---|---|
| | | P3D9 | B8E2 | 31-7-2A | 31-8-5G | 31-12-H3 | 31-9-F4 |
| ATCC 27577 | Homma type 1 | 0 | 0 | 1.1 | 0 | 0 | 0 |
| " 27578 | " 2 | 0 | 1.3 | 0 | 0 | 0 | 0 |
| " 27579 | " 3 | 0 | 0 | 0 | 0 | 0 | 0 |
| " 27580 | " 4 | 0 | 0 | 0 | 0 | 0 | 0 |
| " 27581 | " 5 | 1.6 | 0 | 0 | 0 | 0 | 0 |
| " 27582 | " 6 | 0 | 1.0 | 0 | 1.4 | 0 | 0 |
| " 27583 | " 7 | 0 | 0 | 0 | 0 | 0 | 0 |
| " 27584 | " 8 | 0 | 0 | 0 | 0 | 0 | 1.4 |
| " 27585 | " 9 | 0 | 0 | 0 | 0 | 0 | 0 |
| " 27586 | " 10 | 0 | 0 | 0 | 0 | 1.3 | 0 |
| " 27587 | " 11 | 0 | 0 | 0 | 0 | 0 | 0 |
| Pseudomonas, putida IAM1505 | | 0 | 0 | 0 | 0 | 0 | 0 |
| Pseudomonas, saccharophilia IAM1504 | | 0 | 0 | 0 | 0 | 0 | 0 |
| Eshericbia coli NIHJ-JC-2 | | 0 | 0 | 0 | 0 | 0 | 0 |
| Salmonela minnesota 1114 (smooth) | | 0 | 0 | 0 | 0 | 0 | 0 |
| Proteus mirabilis IFO3489 | | 0 | 0 | 0 | 0 | 0 | 0 |
| Serratia marcescens IAM1184 | | 0 | 0 | 0 | 0 | 0 | 0 |
| Bacillus subtilis ATCC6633 | | 0 | 0 | 0 | 0 | 0 | 0 |
| Staphylococcus aureus 209P | | 0 | 0 | 0 | 0 | 0 | 0 |

From Table 4, it has been made apparent that human monoclonal antibody is specific to P. aeruginosa serotype.

9

Example 5

Determination was made by ELISA to determine how P. aeruginosa human monoclonal antibodies P3D9, B8E2, 31-7-2A, 31-8-5G, 31-9-F4, and 31-12-H3 would bind to P. aeruginosa of the same serotype. The result is shown in Table 5, from which it has been made clear that each of said monoclonal antibodies has reacted with P. aeruginosa of the same serotype up to the highest perfection of 100%.

Table 5

| Monoclonal antibody | Number of P. aeruginosa strains tested | Number of positive bindings (%) |
|---|---|---|
| P3D9 | P. aeruginosa, type 5, 12 strains | 12 (100) |
| B8E2 | P. aeruginosa, type 2, 2 strains | 2 (100) |
| B8E2 | P. aeruginosa, type 7, 7 strains | 7 (100) |
| B8E2 | P. aeruginosa, type 13, 1 strain | 1 (100) |
| 31-7-2A | P. aeruginosa, type 1, 5 strains | 5 (100) |
| 31-8-5G | P. aeruginosa, type 7, 7 strains | 7 (100) |
| 31-9-F4 | P. aeruginosa, type 8, 8 strains | 8 (100) |
| 31-12-H3 | P. aeruginosa, type 10, 6 strains | 6 (100) |

Example 6

Two kinds of mixtures were prepared, one consisting of 0.5 ml of P3D9 with concentration adjusted to 10 $\mu$g/ml and 0.5 ml of P. aeruginosa N-2 (type 5) of 2 X $10^6$ CFU/ml concentration and another consisting of 0.5 ml of the same P3D9 and 0.5 ml of P. aeruginosa O-64 (type 6) of 2 X $10^6$ CFU/ml concentration, and then 0.2 ml of complement of a guinea pig absorbed by each corresponding bacterial strain was added to the respective mixtures. They were cultured at 37°C, during which period 0.3 ml each of samples were taken 30 minutes, 1 hour, and 2 hours after the starting of the cultivation. The samples were plated on HIA medium and the number of colonies was measured. The result is shown in Table 6.

Table 6

| Period of cultivation (min.) | N-2 (CFU/ml) | O-64 (CFU/ml) |
|---|---|---|
| 0 | 1 X $10^6$ | 1 X $10^6$ |
| 30 | 9 x $10^4$ | 9 X $10^5$ |
| 60 | 8 X $10^3$ | 1 x $10^6$ |
| 120 | 2 X 10 | 1 X $10^6$ |
| CFU : Colony forming unit | | |

The above Table 6 shows that P3D9 selectively attached N-2 of type 5 only.

Example 7

Selection of antibody-producing cells and their cell fusion were carried out according to Example 1, wherein human tonsillar cells were used. The obtained hybridoma culture supernatants were transferred to the plates coated with formalin-killed P. aeruginosa and subjected to ELISA wherein goat anti-human IgM antibodies (labelled with alkaline phosphatase) were used as secondary antibodies to give human monoclonal antibodies of IgM type whose serotype reacts with Homma type 5, type 7, and type 8. These human monoclonal antibody-producing hybridomas were cloned twice by limiting dilution and agar dilution to obtain hybridomas productive of human monoclonals of 313-a, 313-b, and 313-c respectively. Thereafter, the obtained hybridomas were partially purified according to the method taken in Example 2 and studied by ELISA to see how they would bind to LPS and heat-treated LPS in order to determine the antigen binding site to which the respective monoclonal antibodies are to react. The result is shown in Table 7.

10

Table 7

| | Type 5 | | Type 7 | | Type 8 | |
|---|---|---|---|---|---|---|
| | LPS | Heat-treated LPS | LPS | Heat-treated LPS | LPS | Heat-treated LPS |
| 313-a | 1.3* | 1.2 | 0 | 0 | 0 | 0 |
| 313-b | 0 | 0 | 0.9 | 0.9 | 0 | 0 |
| 313-c | 0 | 0 | 0 | 0 | 1.1 | 1.0 |

\* Figures in the above table indicate ELISA score 60 minutes after the addition of the substrate solution.

Example 8

Cell fusion was conducted by use of peripheral lymphocytes of a patient with Pseudomonas septicemia according to Example 1 and hybridoma 4H11D10 productive of human IgA monoclonal antibody which specifically reacts only with Homma type 5 was obtained therefrom by screening.

The obtained 4H11D10 reacted with all the nine strains of serotype type 5. The result is shown in Table 8.

Table 8

| Human MCA | Strains belonging to type 5 | | | | | | | | |
|---|---|---|---|---|---|---|---|---|---|
| | O-56 | O-100 | O-101 | C12 | N2 | 1127 | 1237 | 1266 | 2781 |
| 4H11D10 | 0.4* | 0.4 | 0.5 | 0.5 | 0.5 | 0.5 | 0.5 | 0.4 | 0.5 |

\* Figures in the above table indicate ELISA scores 30 minutes after the addition of substrate solution.

Industrial Application

The hybridomas of this invention provide human monoclonal antibodies which specifically bind to LPS, for instance, of P. aeruginosa and these anti-P. aeruginosa antibodies can significantly increase the potency of anti-LPS antibodies in the blood to achieve a curative effect when they are given to patients with P. aeruginosa infections. These monoclonal antibodies also offer applications as reagents and labelling reagents of high accuracy and reliability for testing P. aeruginosa.

**Claims**

1. A mouse-human hybridoma or cell line arising therefrom which produces human anti-P. aeruginosa monoclonal antibodies having specificity for an O-polysaccharide chain of lipopolysaccharide, whose serotype is any of types 1, 2, 5, 7, 8, 10 and 13, wherein said hybridoma is obtained by:
   (a) sensitizing lymphocytes collected from a tonsil or a spleen by mitogen;
   (b) selecting anti-P. aeruginosa antibody-producing human cells;
   (c) fusing said antibody-producing cells with mouse myeloma cells to produce hybridomas;
   (d) identifying and isolating hybridomas which produce said human anti-P. aeruginosa monoclonal antibodies; and
   (e) cloning and culturing said hybridomas obtained in said step (d).

2. A process for producing a mouse-human hybridoma which produces anti-P. aeruginosa human monoclonal antibodies having specificity for an O-polysaccharide chain of lipopolysaccharide, whose serotype is any of types 1, 2, 5, 7, 8, 10 and 13, comprising sensitizing lymphocytes collected from a tonsil or a spleen by mitogen, selecting and collecting tissues which contain many anti-P. aeruginosa antibody-producing cells from among antibody-producing human cellular tissues and then fusing the

11

cells of such human tissues with mouse myeloma cells.

3. The use as a diagnostic reagent of (i) an anti-P. aeruginosa human IgG monoclonal antibody having specificity for an O-polysaccharide chain of lipopolysaccharide, whose serotype is any of types 1, 2, 5, 7, 8, 10 and 13 or of (ii) an IgG monoclonal antibody produced using a hybridoma or cell line as defined in claim 1, the antibody in either case optionally being labelled.

4. A process for producing human anti-P. aeruginosa monoclonal antibody as defined in claim 1, comprising (i) culturing a hybridoma or cell line according to claim 1, or (ii) producing a hybridoma by a process according to claim 2, cloning the hybridoma and culturing it.

5. A process for producing a pharmaceutical formulation, comprising
    (a) producing human anti-P. aeruginosa monoclonal antibody as defined in claim 1 using a hybridoma or cell line according to claim 1, and
    (b) formulating the antibody produced into a pharmaceutical formulation.

## Patentansprüche

1. Maus-Human-Hybridom oder daraus erhaltene Zellinie, die humane, monoklonale Anti-P. aeruginosa-Antikörper mit einer Spezifität für eine O-Polysaccharidkette eines Lipopolysaccharids produzieren, dessen Serotyp einer der Typen 1, 2, 5, 7, 8, 10 und 13 ist, wobei das Hybridom durch folgende Schritte erhalten wird:
    (a) Sensibilisierung von aus Mandeln oder Milz erhaltenen Lymphozyten durch ein Mitogen,
    (b) Selektion auf humane Anti-P. aeruginosa-Antikörper-produzierende Zellen,
    (c) Fusion der Antikörper-produzierenden Zellen mit Maus-Myelomzellen zur Herstellung von Hybridomen,
    (d) Identifizierung und Isolierung von Hybridomen, welche die humanen, monoklonalen Anti-P. aeruginosa-Antikörper produzieren, und
    (e) Klonierung und Kultivierung der in Schritt (d) erhaltenen Hybridomen.

2. Verfahren zur Herstellung eines Maus-Human-Hybridoms, das humane, monoklonale Anti-P. aeruginosa-Antikörper mit einer Spezifität für eine O-Polysaccharidkette eines Lipopolysaccharids produziert, dessen Serotyp einer der Typen 1, 2, 5, 7, 8, 10 und 13 ist, bei dem aus Mandeln oder Milz erhaltene Lymphozyten durch ein Mitogen sensibilisiert, Gewebe mit vielen anti-P. aeruginosa-Antikörper-produzierendenZellen aus humanen, Antikörper-produzierenden Zellgeweben selektioniert und gesammelt und dann die Zellen solcher humaner Gewebe mit Maus-Myelomzellen fusioniert werden.

3. Verwendung eines diagnostischen Reagens von (I) einem humanen, monoklonalen Anti-P. aeruginosa-IgG-Antikörper mit einer Spezifität für eine O-Polysaccharidkette eines Lipopolysaccharid, dessen Serotyp einer der Typen 1, 2, 5, 7, 8, 10 und 13 ist, oder von (II) einem unter Verwendung eines Hybridoms oder einer Zellinie nach Anspruch 1 hergestellten, monoklonalen IgG-Antikörper, wobei der Antikörper in jedem Fall gegebenenfalls markiert ist.

4. Verfahren zur Herstellung eines humanen, monoklonalen Anti-P. aeruginosa-Antikörpers nach Anspruch 1, bei dem (I) ein Hybridom oder eine Zellinie nach Anspruch 1 kultiviert oder (II) ein Hybridom durch ein Verfahren nach Anspruch 2 produziert, das Hybridom kloniert und kultiviert wird.

5. Verfahren zur Herstellung einer pharmazeutischen Formulierung, bei dem
    (a) ein humaner, monoklonaler Anti-P. aeruginosa-Antikörper nach Anspruch 1 unter Verwendung eines Hybridoms oder ein Zellinie nach Anspruch 1 produziert, und
    (b) der produzierte Antikörper zu einer pharmazeutischen Formulierung formuliert wird.

## Revendications

1. Hybridome souris-humain ou ligne cellulaire issue de cet hybridome, qui produit des anticorps monoclonaux humains anti-P. aeruginosa ayant une spécificité pour une chaîne de O-polysaccharide de lipopolysaccharide, dont le sérotype est l'un quelconque des types 1, 2, 5, 7, 8, 10 et 13, l'hybridome précité étant obtenu par :

(a) sensibilisation de lymphocytes recueillis à partir d'une amygdale ou d'une rate par mitogenèse,

(b) sélection de cellules humaines produisant des anticorps anti-P. aeruginosa,

(c) fusion de ces cellules productrices d'anticorps avec des cellules de myélome de souris pour produire des hybridomes,

(d) identification et isolement d'hybridomes qui produisent les anticorps monoclonaux humains anti-P. aeruginosa précités, et

(e) clonage et culture des hybridomes obtenus au stade (d) ci-dessus.

2.  Procédé de production d'un hybridome souris-humain qui produit des anticorps monoclonaux humains anti-P. aeruginosa ayant une spécificité pour une chaîne de O-polysaccharide de lipopolysaccharide, dont le sérotype est l'un quelconque des types 1, 2, 5, 7, 8, 10 et 13, comprenant les étapes consistant à sensibiliser des lymphocytes recueillis à partir d'une amygdale ou d'une rate par mitogenèse, à sélectionner et à recueillir des tissus qui contiennent de nombreuses cellules productrices d'anticorps anti-P. aeruginosa parmi des tissus cellulaires humains producteurs d'anticorps et à fusionner les cellules de ces tissus humains avec des cellules de myélome de souris.

3.  Emploi à titre de réactif diagnostique de (i) un anticorps monoclonal IgG humain anti-P. aeruginosa ayant une spécificité pour une chaîne de O-polysaccharide de lipopolysaccharide dont le sérotype est l'un quelconque des types 1, 2, 5, 7, 8, 10 et 13 ou (ii) d'un anticorps monoclonal IgG produit en utilisant un hybridome ou une ligne cellulaire comme défini(e) dans la revendication 1, l'anticorps de l'un ou l'autre cas étant facultativement marqué.

4.  Procédé de production d'anticorps monoclonaux humains anti-P. aeruginosa selon la revendication 1, comprenant les étapes consistant (i) à cultiver un hybridome ou une ligne cellulaire selon la revendication 1 ou (ii) à produire un hybridome par un procédé selon la revendication 2, à cloner l'hybridome et à le cultiver.

5.  Procédé de production d'une compostion pharmaceutique, comprenant les étapes consistant :

(a) à produire des anticorps monoclonaux humains anti-P. aeruginosa selon la revendication 1 en utilisant un hybridome ou une ligne cellulaire selon la revendication 1, et

(b) à formuler les anticorps produits en composition pharmaceutique.